Europäisches Patentamt

European Patent Office

Office européen des brevets

(11) Publication number: **0 407 063 A1**

# EUROPEAN PATENT APPLICATION

(21) Application number: 90306770.0

(22) Date of filing: 20.06.90

(51) Int. Cl.⁵: **A61B 17/20**

(30) Priority: **06.07.89 US 375910**

(43) Date of publication of application:
**09.01.91 Bulletin 91/02**

(84) Designated Contracting States:
**AT BE CH DE DK ES FR GB GR IT LI LU NL SE**

(71) Applicant: **CONNAUGHT LABORATORIES
LIMITED**
**1755 Steeles Avenue West**
**Willowdale Ontario M2R 3T4(CA)**

(72) Inventor: **Filipski, Ronald J.**
**R.D. 3, P.O. Box 3067A**
**East Stroudsburg, Pennsylvania 18301(US)**
Inventor: **Gilly, John A.**
**Rural Route 2, P.O. Box 289**
**Cresco, Pennsylvania 18326(US)**

(74) Representative: **Burford, Anthony Frederick et
al**
**W.H. Beck, Greener & Co. 7 Stone Buildings**
**Lincoln's InnInn**
**London WC2A 3SZ(GB)**

(54) **Tines structure of clinical applicator.**

(57) A clinical applicator (10) for intradermal or sub-dermal administration of an active substance in a liquid vehicle, such as a vaccine or antigen, has a novel tines structure (26). The tines (26) are provided in the form of an array of eight individual tines (52), each comprising a body member of pyramid shape and having a channel (54) extending from the tip to the base of the body member and having walls at right angles to each other for the length of the channel and to the base. The individual tines (52) are arranged with their apices in a circle and are oriented in two groups of four tines in which the channel of each member forms one corner of a square.

FIG.10B

EP 0 407 063 A1

## TINES STRUCTURE IN CLINICAL APPLICATOR

The present invention is concerned with a novel tines structure in clinical applicators for the application of liquid antigen or vaccine to the intradermal or subdermal region of skin.

The intradermal or subdermal application of liquid antigen or vaccine using scarifying devices having a plurality of tines or needles, usually to the forearm for testing or vaccination, particularly in children, is well known.

Not a great deal of attention has been paid to the design of the tines and, as a result, inefficient use of liquid antigen or vaccine results. The purpose of the present invention is to provide a design of tine which achieves the desired intradermal or subdermal administration of the antigen or vaccine in a highly efficient manner.

A search in the facilities of the United States Patent and Trademark Office has located the following U.S. Patents as the closest prior art.

U.S. 3,074,403 Cooper et al
U.S. 3,322,121 Banker
U.S. 3,688,764 Reed
U.S. 3,905,371 Stickl et al
U.S. 4,109,655 Chacornac

As will be seen from the description below, none of these prior patents describe the novel tine structure of the present invention.

In accordance with one aspect of the present invention, there is provided a novel tine structure for a clinical applicator. The tine comprises a body member of pyramid shape having a channel formed in one face thereof extending from the tip to the base of the body member. The walls of the channel are at right angles to each other for the length of the channel and at right angles to the base.

In accordance with another aspect of the present invention, there is provided an improvement in a clinical applicator having a plurality of tines for intradermal or subdermal administration of an active substance in a liquid vehicle. The improvement lies in an array of eight individual ones of the tines defined above. The tines are arranged in the array having their apices arranged in a circle and being oriented in two group of four tines in which the channel of each member forms one corner of a square.

In one embodiment, a further tine may be provided comprising a body of pyramid shape and located with its apex at the center formed by the apices of the array of eight individual tines.

The tine structure of the present invention contrasts markedly with that illustrated in the prior art described above. For example, U.S. Patent no. 3,074,403 describes a device intended for the delivery of dried materials, the tines being dipped in liquid and then dried. In contrast, in the present invention, the tines are designed for the delivery of liquid product.

The capillary action which is attributed to the design in U.S. Patent no. 3,074,403 arises from an edge space between individual tine members, which itself results from stamping out metal, while the capillary action in the present invention arises from the channel in the one-piece molded tine element.

Further, the structure of the tines in the device of U.S. Patent no. 3,074,403 results, in use, in two single slit punctures into the skin carrying dried antigen, whereas in the present invention, the action of the tines is to form a channel in the punctured skin to facilitate the flow of liquid antigen into the skin.

U.S. Patent no. 4,109,655 describes a scarifying device which employs tines having a star-shaped cross-section. In the tine structure of the present invention, a groove is formed by a channel which runs from the tip of the tine to the base where the channel surfaces are perpendicular to the base, which enables antigen to be transferred along the tine and into the skin through an opening which has equal proportion from base to tip. In contrast, in U.S. Patent no. 4,109,655, channel surfaces meet the base at an angle, so that, a channel in the skin, if made initially, would not enlarge and would allow minimal or no flow of antigen to the skin.

The frictional forces for retention of liquid applied to the tines are greater in the present invention. In the prior art, the angle of tine inner surface is 120° and, as a consequence, the tine would lose liquid material on it if force towards the tip is applied.

We have previously described in European patent application No. 90300890.2 filed January 29, 1990 a novel structure for a clinical applicator, which provides for a visual indication of the location of administration of the active substance. The tines structure defined herein has particular application to a clinical applicator of the type described in our prior application, but may be used with any convenient applicator for liquid antigen or vaccine.

The antigens or vaccines which may be administered using the device of the present invention may be one of a variety of materials known to be administered using similar multi-tine devices, including tuberculin tests, tetanus sensitivity, diagnosis of histoplasmosis, blastomycosis, coccidimycosis, cryptococcisis, sporotrichosis, allergen sensitivity and smallpox vaccination.

The invention is described further, by way of illustration, with reference to the accompanying drawings in which:

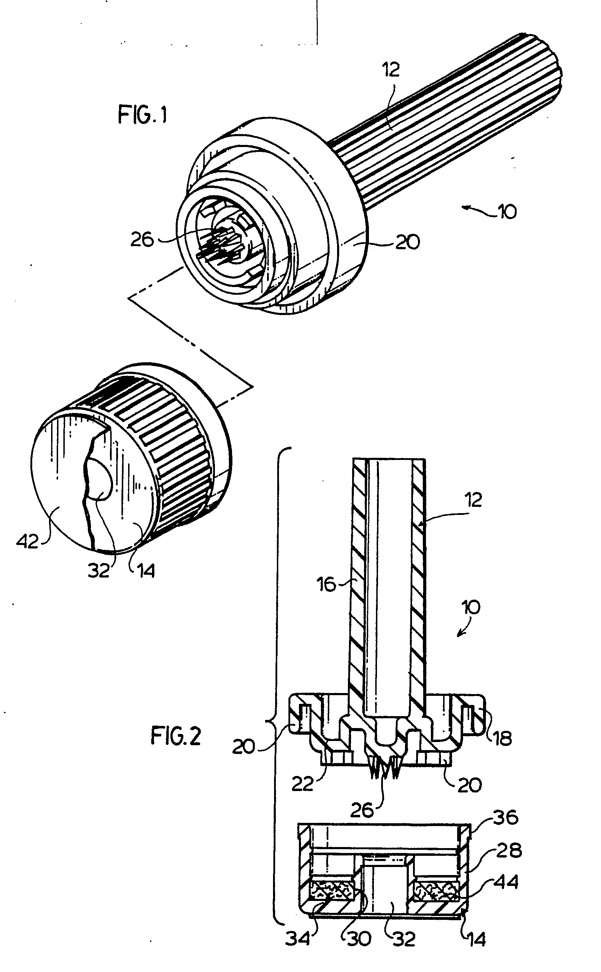

Figure 1 is a perspective view of one form of clinical applicator or scarifying device, with the cap member removed from the inoculator member, employing an arrangement of tines in accordance with one embodiment of the invention;

Figure 2 is a sectional view of the clinical applicator of Figure 1;

Figure 3 is a perspective view of the clinical applicator of Figure 1, with the cap member assembled with the inoculator member;

Figure 4 is a sectional view of the clinical applicator of Figure 3;

Figure 5 is a sectional view of the inoculator member during administration of liquid antigen or vaccine to skin;

Figure 6 is a perspective view of the inoculator member and skin showing the visual indication of the location of administration of the active substance;

Figure 7 is a close-up perspective view of the arrangement of tines;

Figure 8 is a perspective view of the tines of one embodiment of the invention;

Figure 9 is a plan view of the tines of Figure 8; and

Figures 10A and 10B are close-up perspective views of the structure of two different tines used in the arrangement of Figure 8.

Referring to the drawings, a scarifying device 10 comprises an inoculator member 12 and a cap member 14. The inoculator member 12, which is a one-piece molded part, comprises an elongate handle 16 in the form of a hollow tube and a head 18 having a cylindrical outer channel structure 20 joined to the handle 16 by a radially-directed flange or wall 22.

A series of projections 24 extend from the wall 22 in the shape and form of an identifying location indicating marking. A plurality of parallel needles or tines 26 protrude from the handle 16 beyond the projections 24 to effect intradermal or subdermal application of an active substance.

The cap member 14, also a one-piece molded part, comprises an outer wall 28, a parallel inner wall 30 defining a central opening 32 and a radially-directed wall 34. To enable the cap member 14 to be releasably mounted to the inoculator member 12, the outer wall 28 is provided with an integral protrusion 36 at its upper extremity, which snap-locks into a corresponding recess formed in the channel member 20 when the outer wall 28 is received in the channel.

At the same time, the inner wall 30 bears against a central cylindrical protrusion 38 on which the tines 26 are mounted. In this way, the opening 32 into which the tines 26 protrude is sealed off.

The cap 14 is assembled with the handle 12. With the assembly in a vertical orientation, an active liquid material 40 is placed in the opening 32 and a foil covering 42 is adhered over the open end of the opening 32 to hermetically seal the active substance in the cavity into which project the needles 26.

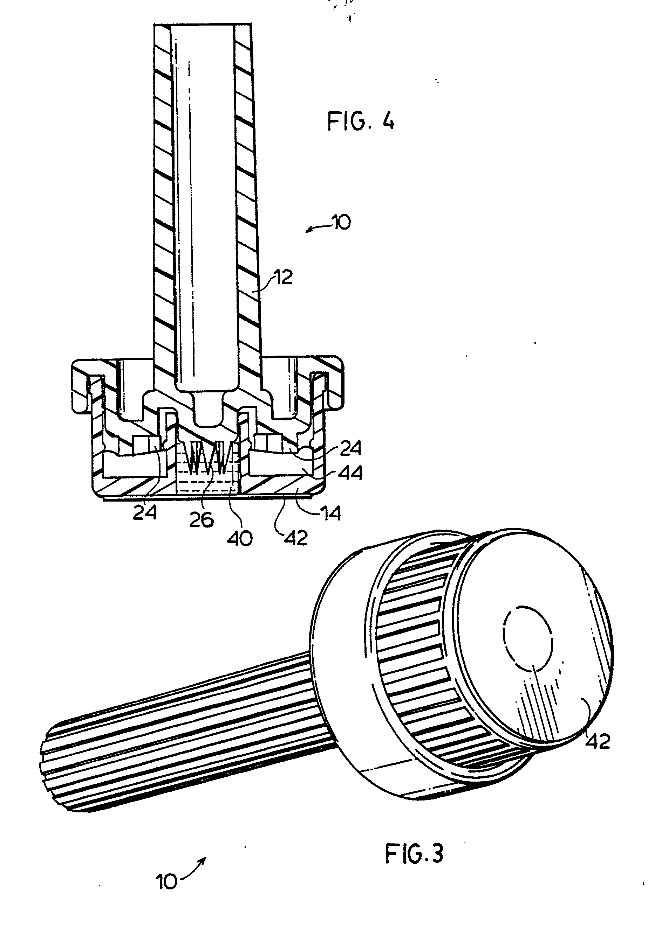

The cap member 14 also includes a ring 44 of absorbent material which is seated against the inner surface of the wall 34 and which has a marking fluid absorbed therein, such as a dye or ink which, when dry, resists removal by washing. When the cap member 14 is assembled to the handle member 12, the projections 24 engage the ring 44, as seen in Figure 4.

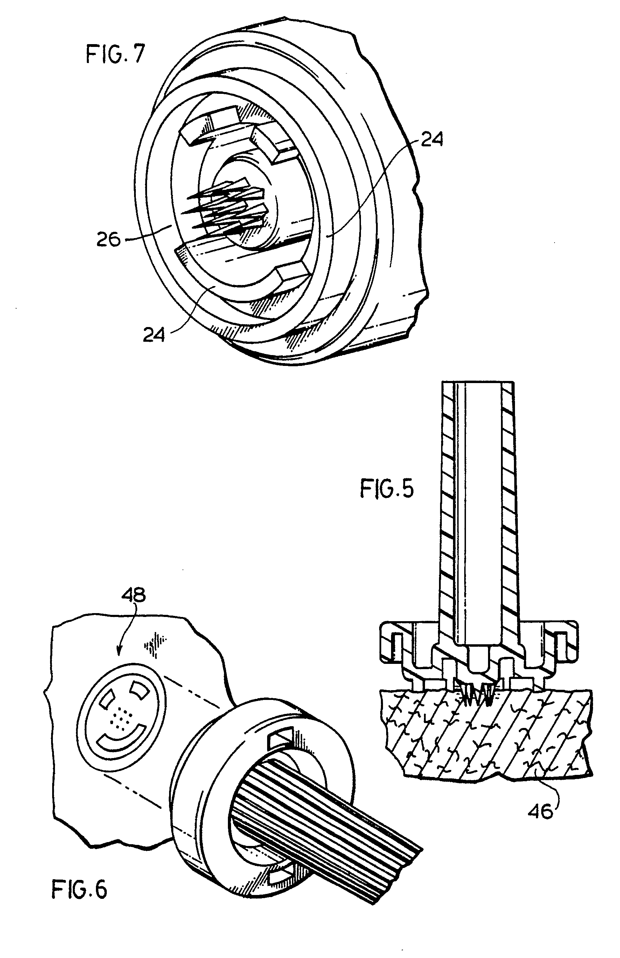

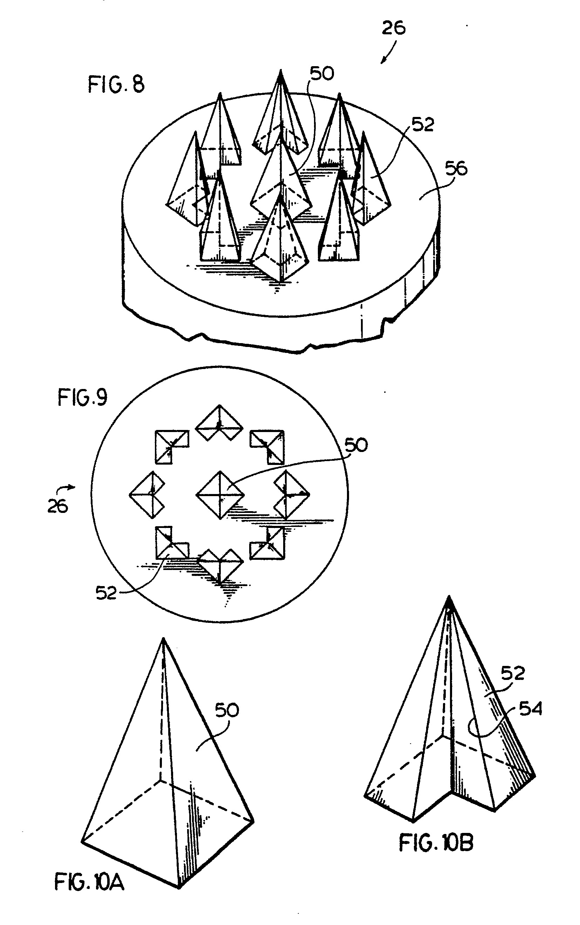

When it is desired to make an application of the active material 40 to a patient's skin, the inoculator member 12 is removed from the cap member 14 and the head 18 is brought into contact with the skin 46 of the patient. The needles 26, which are wetted with the active material 40, penetrate the skin 46 and make an intradermal or subdermal application of the active material, depending on the length of the needles 26. At the same time, the projections 24, which are wetted with marking fluid from contact with the ring 44, engage the outer surface of the skin, as seen in Figure 5, and form a pattern on the skin corresponding to the pattern of the projections 24, as seen in Figure 6.

The pattern 48 of marking fluid, a "happy face" in the illustrated embodiment, thereby provides a visual indication of the location of the intradermal or subdermal application but does not, in any way, interfere with the application site. For adult use, the visual image may take the form of a simple ring.

Turning now to consideration of Figures 7 to 10, wherein there is illustrated the tines structure 26 of the present invention in more detail. As may be seen, the tines structure comprises a central tine 50 which is in the shape of a pyramid and a circular array of eight tines 52 which are each in the shape of a channelled pyramid.

Each of the tines 52 has a groove 54 therein which runs from the tip of the tines to the base and has surfaces which are perpendicular to the support surface 56 and to each other. Each of the complete faces is in the form of an isosceles triangle having an apex angle of about 5° to about 20°.

The tines 52 are arranged with their apices in a circle, the center of which is the apex of the tine 50. The tines 52 are arranged in two groups of four, each made up of an alternate one of the tines 52, in which the channels 54 define the corners of a square.

When the tines penetrate the skin, the channels 54 enable liquid antigen or vaccine to be transferred along the tine and into the skin through an opening which has an equal proportion from base to tip.

The 90° angle between the walls of the channel 54 inhibits skin elasticity from closing the channel, enabling efficient administration of the liquid to be effected.

The individual tines, 50, 52 may be metal but preferably are of plastic material integrally molded with the remainder of the innocular member 12.

In summary of this disclosure, the present invention provides a novel form of tines structure which enables efficient administration of liquid antigen or vaccine to be effective.

## Claims

1. A tine for a clinical applicator, characterized by a body member (52) of pyramid shape having a channel (54) extending from the tip to the base of said body member (52) and having walls at right angles to each other for the length of the channel and at right angles to the base.

2. A tine as claimed in Claim 1, wherein said channel (54) is at a corner of the body member (52).

3. A tine as claimed in Claim 1 or Claim 2, wherein the body member (52) has a square base.

4. A tine as claimed in any one of the preceding claims, wherein the body member (52) is a regular pyramid.

5. A tine as claimed in Claim 4, in which each complete face of said body member (52) is in the form of an isosceles triangle having an apex angle of 5° to 20°.

6. A clinical applicator having a plurality of tines (26) for intradermal or subdermal administration of an active substance in a liquid vehicle, characterized in that at least some of said tines (26) are as claimed in any one of the preceding claims.

7. A clinical applicator as claimed in Claim 6, wherein eight of said tines (26) are arranged in an array with their apices disposed in a circle and are orientated in two groups of four tines in which the channel (54) of each member forms one corner of a square.

8. A clinical applicator as claimed in Claim 7, wherein a further tine (50) comprising a body of pyramid shape is located with its apex at the center of said circle.

9. A clinical applicator as claimed in Claim 8, wherein said further tine (50) is identical with the body member (52) of each of the channel tines but has no channel (54) therein..

10. A clinical applicator as claimed in any one of Claims 6 to 9, comprising (a) an inoculator member (12) having an elongate handle (16), said array of tines (26) extending in parallel manner axially from one end of said elongate handle (16) for penetration of skin (46) for administration of the liquid antigen or vaccine (40) on said tines (26) to an intradermal or subdermal location, and a generally U-shaped channel member (20) integrally formed with said one end of said elongate handle (16); and (b) a cap member (14) comprising an outer ring member (28) having means (36) to releasably assemble the cap member (14) with the inoculator member (12), and an axially-inwardly directed recess closed at one end and arranged to house said liquid antigen or vaccine (40) and to receive said tine (26) therein when said cap member (14) is assembled with said inoculator member (12).

FIG.1

FIG.2

FIG. 4

FIG.3

FIG. 7

24

26

24

FIG.5

48

46

FIG.6

FIG. 8

FIG. 9

FIG. 10A

FIG. 10B

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-3 246 647  (TAYLOR)<br>* Column 4, lines 5-8; figure 9 *<br>--- | 1 | A 61 B   17/20 |
| A | FR-A-2 535 602  (GUERIN)<br>* Page 6, line 28 - page 7, line 2; figures 3-5 *<br>--- | 1 | |
| A | US-A-3 512 520  (COWAN)<br>--- | | |
| A | FR-A-2 150 994  (SCHWEIZ, SERUM- & IMPFINSTITUT UND INSTITUT ZUR ERFORSCHUNG DER INFEKTIONSKRANKHEITEN)<br>----- | | |

TECHNICAL FIELDS
SEARCHED (Int. Cl.5)

A 61 B
A 61 D
A 01 K

The present search report has been drawn up for all claims

| Place of search | Date of completion of the search | Examiner |
|---|---|---|
| THE HAGUE | 11-10-1990 | GLAS J. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P0401)